# FASCICULE DE BREVET EUROPEEN

(11) **EP 2 519 233 B1**
(45) Date de publication et mention de la délivrance du brevet: **17.12.2014**
(21) Numéro de dépôt: 10808937.6
(22) Date de dépôt: 29.12.2010
(51) Int. Cl.: A61K 31/18, A61K 31/451, A61K 31/517, A61K 45/06, A61P 25/32

(54) **COMPOSITION PHARMACEUTIQUE POUR LE TRAITEMENT DE LA DÉPENDANCE À L'ALCOOL**
PHARMAZEUTISCHE ZUSAMMENSETZUNG ZUR BEHANDLUNG VON ALKOHOLABHÄNGIGKEIT
PHARMACEUTICAL COMPOSITION FOR TREATING ALCOHOL DEPENDENCY

(30) Priorité: 30.12.2009 FR 0959669
(43) Date de publication de la demande: 07.11.2012
(73) Titulaire: Greenpharma, 63360 Saint Beauzire (FR); Key Obs, 45100 Orleans (FR)
(72) Inventeur: BERNARD, Philippe, F-45240 La Ferté Saint Aubin (FR); TROVERO, Fabrice, F-41500 Avaray (FR)
(74) Mandataire: Verriest, Philippe
(86) Numéro de dépôt international: PCT/FR2010/052932
(87) Numéro de publication internationale: WO 2011/080488

(56) Documents cités:
- WO-A1-2006/018538
- ESTEBAN M M ET AL: "Reduced ethanol consumption during cyproheptadine administration in rats from a long-term alcohol-treated colony", PHYSIOLOGY AND BEHAVIOR, ELSEVIER SCIENCE LTD., OXFORD, GB, vol. 38, no. 2, 1 janvier 1986 (1986-01-01), pages 247-254, XP024315292, ISSN: 0031-9384 [extrait le 1986-01-01]
- SIMPSON T L ET AL: "A PILOT TRIAL OF THE ALPHA-1 ADRENERGIC ANTAGONIST, PRAZOSIN, FOR ALCOHOL DEPENDENCE", ALCOHOLISM CLINICAL AND EXPERIMENTAL RESEARCH, vol. 33, no. 6, Sp. Iss. S1, juin 2009 (2009-06), page 312A, XP002575138, & 32ND ANNUAL SCIENTIFIC MEETING OF THE RESEARCH-SOCIETY-ON-ALCOHOLISM; SAN DIEGO, CA, USA; JUNE 20 -24, 2009 ISSN: 0145-6008 cité dans la demande
- WALKER ET AL: "alpha1-noradrenergic receptor antagonism blocks dependence-induced increases in responding for ethanol", ALCOHOL, PERGAMON PRESS, LONDON, GB, vol. 42, no. 2, 20 mars 2008 (2008-03-20), pages 91-97, XP022550474, ISSN: 0741-8329

## Description

La présente invention a pour objet une composition pharmaceutique utile pour le traitement de la dépendance à l'alcool.

Le traitement pharmacologique des phénomènes de dépendance, parmi lesquels la dépendance à l'alcool, est un enjeu majeur de santé publique. Pendant très longtemps, le traitement des dépendances aux différentes substances addictives telles que l'alcool s'est principalement orienté vers l'aide au sevrage, c'est-à-dire l'arrêt de la consommation. Cette stratégie thérapeutique s'est donc concentrée sur la réduction des symptômes physiques qui traduisent l'état de manque au moment de cet arrêt. Ces symptômes, qui marquent la dépendance physique, sont fonction des produits consommés : dans le cadre de la dépendance à l'alcool, ils se traduisent par des tremblements. Le sevrage déclenche également des troubles du comportement (anxiété, irascibilité, angoisse, agitation...).

Dans le cadre du traitement des toxicomanies, la pharmacopée actuelle s'est donc développée autour des produits de substitution dont l'objectif thérapeutique principal est de limiter les symptômes physiques induits par le sevrage. Pour autant, il existe peu voire pas de substances visant à contrecarrer les états de dépendance psychique, c'est à dire le besoin irrépressible (ou « craving ») d'alcool. Cet état de dépendance psychique est beaucoup plus robuste et est la plupart du temps à l'origine des rechutes.

A la date de la présente invention, des traitements pharmacologiques ont été ou sont proposés dans le cadre du traitement de la dépendance à l'alcool.

Parmi ces traitements, on peut citer l'utilisation d'antagonistes opiacés qui stimulent l'activité des systèmes dopaminergiques. Les systèmes opioïdes jouent un rôle clef dans l'apparition de la dépendance à l'alcool en contribuant aux effets euphorisants et appétitifs de la prise d'alcool.

La naltrexone, un antagoniste opiacé, a été testée dans des essais cliniques. Les études ont alors montré que la naltrexone diminuait la prise d'alcool, la fréquence de rechute et le désir de boire, en particulier dans le cas d'alcoolisation grave. C'est donc le premier agent pharmacologique contre l'alcoolisme qui agit autrement qu'en déclenchant un phénomène d'aversion.

Malheureusement, l'usage de la naltrexone est limité en raison de ses effets secondaires au niveau gastro-intestinal (nausées, vomissements, baisse de l'appétit) (O'Malley et al., 1992, Naltrexone and coping skills therapy for alcohol dependence, A controlled study, Arch Gen Psychiatry; 1992, 49 ; p 881-7 ; Volpicelli et al., Naltrexone in the treatment of alcohol dependence, Arch Gen Psychiatry, 1992, 49, p 876-80 ; Kranzler et al., Naltrexone vs. Nefazodone for Treatment of Alcohol Dependence -A Placebo-Controlled Tribal ; Neuropsychopharmacology, 2000, 22, 5, p 493-503).

On peut également citer l'utilisation du naltrindole, un antagoniste des récepteurs opiacés type δ, qui a montré une certaine efficacité dans les modèles animaux.

L'utilisation de l'acamprosate a également été envisagée. Même si son mécanisme d'action n'est pas totalement élucidé, un faisceau d'arguments permet de penser que l'action de l'acamprosate passe par une modulation de la transmission glutamatergique. Il semblerait que la molécule soit efficace, au moins dans le traitement des symptômes de sevrage. Son efficacité vis-à-vis de l'appétence à l'alcool est encore débattue.

Les antidépresseurs sérotoninergiques ont également été utilisés. La transmission sérotoninergique joue un rôle important dans la pathophysiologie de la dépendance alcoolique. Les inhibiteurs de la recapture de sérotonine sont des antidépresseurs qui ont été testés dans le traitement de l'alcoolisme (Naranjo et al., Zimelidine-induced variations in alcohol intake by nondepressed heavy drinkers ; Clin Pharmacol Ther, 1984 35, p374-81 ; Naranjo et al., The serotonin uptake inhibitor citalopram attenuates ethanol intake, Clin Pharmacol Ther, 1987, 41, p 266-74 ; Naranjo et al., Fluoxetine differentially alters alcohol intake and other consummatory behaviors in problem drinkers, Clin Pharmacol Ther, 1990, 47, p 490-8). Les essais thérapeutiques à partir de ces substances sérotoninergiques ont donné des résultats variables, les études cliniques ne mettant pas en évidence de réelle efficacité.

On peut citer l'utilisation des benzodiazépines, médicaments les plus utilisés pour le sevrage alcoolique (Lejoyeux et al. Benzodiazepine treatment for alcohol-dependent patients, Alcohol & Alcoholism, 1998, Vol. 33. No.6. pp. 563-575). Les benzodiazépines sont utilisées avec efficacité au moment du sevrage. Cette efficacité est discutée à long terme, et ce d'autant plus que les patients poursuivent ce type de traitement sur de longues périodes pour lutter contre des symptômes de l'abstinence tels que l'anxiété et l'insomnie. La question du rapport bénéfice/risque se pose, dans la mesure où il s'agit de remplacer un produit d'abus par un autre chez un patient déjà sensible aux phénomènes de dépendance.

Enfin, on peut citer l'utilisation des médications aversives. Ainsi, le premier médicament aversif contre l'alcool fut le disulfirame, utilisé depuis 1940. Lorsqu'il est consommé simultanément avec de l'alcool, ce produit déclenche des effets désagréables tels que des nausées, des vomissements, une augmentation de la pression artérielle et du rythme cardiaque.

A la date de la présente invention, il demeure donc nécessaire de mettre au point de nouveaux traitements pour lutter contre la dépendance à l'alcool.

La cyproheptadine désigne la 4-(5 H-dibenzo [a,d]cyclohepten-5-ylidene)- 1 - methylpiperidine hydrochloride de formule :

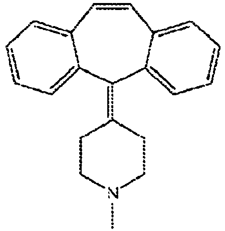

Ce principe actif est connu pour avoir une action antagoniste sur les récepteurs 5-HT2 serotoninergiques et est habituellement utilisé comme antihistaminique/anticholinergique et antiserotonergique. Commercialisé sous le nom de Périactine®, il est recommandé pour le traitement symptomatique des manifestations allergiques diverses.

La prazosine désigne la 2-[4-(2-furoyl)piperazin-1-yl]-6,7-dimethoxyquinazolin-4-amine de formule :

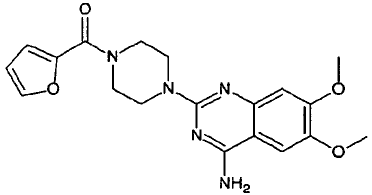

Ce principe actif est connu pour avoir une action antagoniste sur les récepteurs alphal-noradrénergiques et est habituellement utilisé comme agent hypotenseur. Commercialisé sous le nom de Minipress®, il est recommandé pour traiter l'hypertension artérielle, et l'insuffisance ventriculaire gauche congestive, mais également dans le cadre du traitement symptomatique des phénomènes de Raynaud (primitifs ou secondaires) et de certaines manifestations fonctionnelles liées à l'hypertrophie bénigne prostatique.

La possible utilisation de la prazosine seule pour le traitement de la dépendance à l'alcool a été rapportée par Tracy L. Simpson et al. dans A Pilot Trial of the Alpha-1 Adrenergic Antagonist, Prazosine, for Alcohol Dependence, Alcoholism : Clinical and Experimental Research, Vol.32, No.11, November 2008, pp.1-9. Cependant, dans le cadre de cette étude, les doses de prazosine administrées ont été de 4 mg le matin, 4 mg le midi et 8 mg le soir, soit une dose journalière de 16 mg.
Une telle dose est largement supérieure à la dose thérapeutique maximum recommandée soit 10 mg de prazosine par jour, et est de plus très proche de la dose maximum à ne pas dépasser, soit 20 mg par jour. A ces doses apparaissent les premiers effets secondaires tels que vertige orthostatique, fatigue et somnolence. D'autre part, les auteurs eux-mêmes font part de leurs réserves au sujet des résultats obtenus au cours de cette étude clinique du fait de la faiblesse des effectifs (7 patients traités) et de la courte durée de l'essai clinique (6 semaines) au regard de la recherche d'un bénéfice thérapeutique sur le phénomène de dépendance qui se juge principalement sur les risques de rechute à moyen et court termes. Chez l'homme, la période critique de rechute est en effet d'au moins une année d'abstinence.

L'alfuzosine désigne la N-[3-[(4-amino-6,7-dimethoxy-quinazolin-2-yl)- methyl-amino]propyl] tetrahydrofuran- 2-carboxamide de formule :

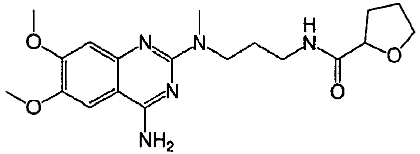

Ce principe actif est connu pour avoir une action antagoniste sur les récepteurs alpha1-noradrénergiques. Commercialisé sous le nom d'Urion®, il est recommandé pour traiter les symptômes fonctionnels de l'hypertrophie bénigne de la prostate.

La térazosine désigne la 6,7-dimethoxy-2-[4-(tetrahydrofuran-2-ylcarbonyl)piperazin-1-yl]quinazolin-4-amine de formule :

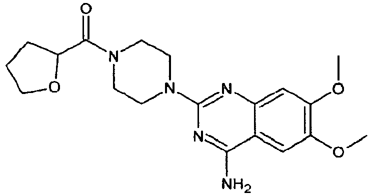

Ce principe actif est connu pour avoir une action antagoniste sur les récepteurs alpha1-noradrénergiques. Commercialisé sous le nom d'Hytrine® ou Dysalfa®, il est recommandé pour traiter les symptômes fonctionnels de l'hypertrophie bénigne de la prostate.

La tamsulosine désigne le (R)-5-(2-(2-(2-ethoxyphenoxy)ethylamino)propyl)-2-methoxybenzenesulfonamide de formule :

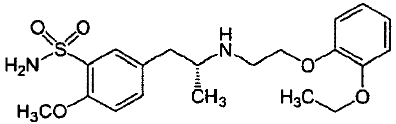

Ce principe actif est connu pour avoir une action antagoniste sur les récepteurs alpha1-noradrénergiques. Commercialisé sous le nom de Josir® ou Emix®, il est recommandé pour traiter les symptômes fonctionnels de l'hypertrophie bénigne de la prostate.

La demande de brevet WO 2006/018538 décrit l'utilisation d'associations de principes actifs ayant une action antagoniste simultanée sur les récepteurs alpha1-noradrénergiques, NMDA glutamatergiques et 5-HT2 sérotoninergiques pour traiter les pathologies du système nerveux central telles que les pharmaco-dépendances, les psychoses, le tabagisme, les troubles liés à la consommation d'alcool, la schizophrénie, les états psychotiques aigus et chroniques, les démences, les troubles de l'humeur, les troubles de l'attention, les troubles du sommeil, les troubles de l'impulsivité, l'hyperactivité, les états psychotiques aigus et chroniques, les états de dépendance aux substances addictives, la dépendance à l'alcool, la dépendance aux psychostimulants, la dépendance aux opiacés, la dépendance aux benzodiazépines, la dépendance au tabac et la dépendance aux jeux. La composition comprenant l'ifenprodil et la cyproheptadine est préférée et a démontré une activité dans le cadre du traitement de la dépendance à l'amphétamine.
Les compositions décrites et testées dans de la cadre de cette demande de brevet doivent avoir une action antagoniste simultanée sur trois récepteurs : les récepteurs alpha1-noradrénergiques, les récepteurs NMDA glutamatergiques et les récepteurs 5-HT2 sérotoninergiques.
Or, une action antagoniste simultanée sur ces trois récepteurs augmente les risques d'apparition d'effets secondaires majeurs, en particulier lors de traitements prolongés.

Il y a donc une nécessité d'identifier de nouvelles compositions pharmaceutiques ayant une activité thérapeutique au moins équivalente aux compositions déjà connues mais permettant de limiter les risques d'apparition d'effets secondaires majeurs.

Il a maintenant été trouvé, de façon toute à fait inattendue, que certains composés ayant une action antagoniste sur les récepteurs alpha1-noradrénergiques pouvaient être utilisés en combinaison avec un composé ayant une action antagoniste sur les récepteurs 5-HT2 sérotoninergiques dans le cadre du traitement de la dépendance à l'alcool avec une efficacité au moins comparables, voire meilleure, que les compositions décrites dans l'art antérieur. D'autre part, une synergie d'activité a également été observée lors de l'utilisation combinée de certains composés ayant une action antagoniste sur les récepteurs alpha1-noradrénergiques pouvaient être utilisés en combinaison avec un composé ayant une action antagoniste sur les récepteurs 5-HT2 sérotoninergiques dans le cadre du traitement de la dépendance à l'alcool en comparaison de l'activité de chacun des composés utilisés seuls.

Ainsi, la présente invention a pour objet une composition pharmaceutique pour le traitement de la dépendance à l'alcool chez l'être humain comprenant deux principes actifs :
- un composé ayant une action antagoniste sur les récepteurs 5-HT2 sérotoninergiques choisi comme étant la cyproheptadine ; et
- un composé ayant une action antagoniste sur les récepteurs alpha1-noradrénergiques choisi parmi la prazosine, l'alfuzosine, la térazosine et la tamsulosine.

La composition pharmaceutique selon l'invention permet de limiter les risques d'apparition d'effets secondaires majeurs en ayant une action antagoniste simultanée sur les récepteurs alpha1-noradrénergiques et 5-HT2 sérotoninergiques, tout en maintenant une efficacité au moins comparable à celle des compositions pharmaceutiques connues pour avoir une action antagoniste simultanée sur les récepteurs alpha1-noradrénergiques 5-HT2 sérotoninergiques et NMDA glutamatergiques. De plus, la composition selon l'invention a mis en évidence une synergie inattendue entre les principes actifs.

Dans le cadre de la présente invention :
- on entend par « administration journalière » une administration une fois par jour ou une administration une fois par 24 heures ; et
- on entend par « calendrier continu » le traitement thérapeutique continu d'un patient, comprenant l'administration successive d'une ou plusieurs compositions thérapeutiques (dont multithérapies, selon l'invention ou non), identiques ou différentes, chacune avec son propre schéma d'administration thérapeutique (nombre d'administrations journalières et nombre de jours d'administration sur une période donnée, semaine par exemple) et ce, de façon illimitée et non séquencée ou espacée dans le temps, c'est-à-dire sans interruption de traitement ;
- on entend par «sel pharmaceutiquement acceptable » d'un principe actif tout sel d'addition dudit principe actif avec un acide minéral ou organique par action d'un tel acide au sein d'un solvant organique ou aqueux tel qu'un alcool, une cétone, un éther ou un solvant chloré, et qui soit acceptable d'un point de vue pharmaceutique ;
- on entend par « dérivé pharmaceutiquement acceptable » d'un principe actif tout « prodrogue » **ou** « métabolite » dudit principe actif, ainsi que leur sel pharmaceutiquement acceptable ;
- on entend par « prodrogue » d'un principe actif tout composé dont la biotransformation dans l'organisme aboutit audit principe actif ;
- on entend par « métabolite » d'un principe actif tout produit intermédiaire résultant de la transformation dudit principe actif dans l'organisme lors d'un processus métabolique ;
- cyproheptadine désigne le (5 H-dibenzo [a,d]cyclohepten-5-ylidene)- 1 - methylpiperidine hydrochloride, ainsi que ses sels ou dérivés pharmaceutiquement acceptables ;
- prazosine désigne la 2-[4-(2-furoyl)piperazin-1-yl]-6,7-dimethoxyquinazolin-4-amine, ainsi que ses sels ou dérivés pharmaceutiquement acceptables ;
- alfuzosine désigne la N-[3-[(4-amino-6,7-dimethoxy-quinazolin-2-yl)- methyl-amino]propyl] tetrahydrofuran- 2-carboxamide, ainsi que ses sels ou dérivés pharmaceutiquement acceptables ;
- térazosine désigne la 6,7-dimethoxy-2-[4-(tetrahydrofuran-2-ylcarbonyl)piperazin-1-yl]quinazolin-4-amine, ainsi que ses sels ou dérivés pharmaceutiquement acceptables ; et - **tamsulosine** désigne la (R)-5-(2-(2-(2-ethoxyphenoxy)ethylamino)propyl)-2-methoxybenzenesulfonamide, ainsi que ses sels ou dérivés pharmaceutiquement acceptables.

Préférentiellement, la présente invention a pour objet une composition pharmaceutique telle que définie ci-dessus, dans laquelle le composé ayant une action antagoniste sur les récepteurs alpha 1-noradrénergiques est la prazosine.

La composition pharmaceutique selon la présente invention contient les principes actifs en quantité suffisante pour assurer l'effet thérapeutique souhaité, c'est-à-dire le traitement de la dépendance à l'alcool chez le patient traité.

De préférence, les quantités journalières suivantes de principes actifs sont utilisées pour préparer la composition pharmaceutique selon l'invention :
- de 0,04 à 20 mg de cyproheptadine, de préférence de 0,4 à 10 mg de cyproheptadine ;
- de 0,025 à 20 mg de prazosine, de préférence de 0,25 à 10 mg de prazosine ;
- de 0,075 à 10 mg d'alfuzosine, de préférence de 0,75 à 7,5 mg d'alfuzosine ;
- de 0,01 à 5 mg de térazosine, de préférence de 0,1 à 2,5 mg de térazosine ;
- de 0,004 à 0,4 mg de tamsulosine, de préférence de 0,04 à 0,4 mg de tamsulosine.

La composition pharmaceutique selon la présente invention peut être formulée sous toute forme galénique nécessaire à son administration. En particulier, s'agissant d'administration par voie orale, les compositions selon la présente invention peuvent être formulées sous forme de comprimés enrobés ou non, effervescents, solubles, orodispersibles, gastrorésistants ou à libération modifiée ; de dragées ; de capsules à enveloppe dure (ou gélules) ; de capsules à enveloppe molle ; de granules ; de granulés ; de pilules ; de pastilles. S'agissant d'administration par voie nasale, la composition peut être formulée sous forme de spray ou de poudre à inhaler. S'agissant d'administration par voie systémique, la composition selon l'invention peut être formulée sous forme de poudre lyophilisée stérile pour injection. Les compositions pharmaceutiques selon la présente invention pourront donc comprendre, en plus des principes actifs, tout adjuvant de formulation pharmaceutiquement acceptable, connu de l'homme du métier et qui est nécessaire à la préparation de la composition pharmaceutique sous la forme souhaitée.

La composition pharmaceutique selon l'invention peut être administrée de une à quatre fois par jour, sans dépasser la dose journalière maximum. Ainsi, la présente invention a également pour objet une composition pharmaceutique telle que définie précédemment pour une administration 1 à 4 fois par jour au patient dépendant à l'alcool

La composition pharmaceutique selon l'invention peut être administrée à tout moment de la journée, avant, pendant ou après les repas, sans que cela n'influe sur l'efficacité du traitement.

La composition pharmaceutique selon la présente invention peut être administrée au patient une ou plusieurs fois par semaine. Ainsi, la présente invention a également pour objet une composition pharmaceutique telle que définie précédemment pour une administration journalière 1 à 7 jours par semaine au patient dépendant à l'alcool.

La composition selon la présente invention peut être administrée selon un calendrier continu.

Un autre objet de la présente invention concerne également l'utilisation d'une composition pharmaceutique telle que définie précédemment pour la préparation d'un médicament destiné au traitement de la dépendance à l'alcool chez l'être humain.

Un autre objet de la présente invention concerne également l'utilisation d'une composition pharmaceutique telle que définie précédemment pour la préparation d'un médicament destiné au traitement de la dépendance à l'alcool chez l'être humain, ledit médicament étant administré 1 à 4 fois par jour.

Un autre objet de la présente invention concerne également l'utilisation d'une composition pharmaceutique telle que définie précédemment pour la préparation d'un médicament destiné au traitement de la dépendance à l'alcool chez l'être humain, ledit médicament étant administré de façon journalière 1 à 7 jours par semaine.

La présente invention a également pour objet des moyens pour traiter la dépendance à l'alcool chez un être humain par l'administration d'une composition pharmaceutique telle que de définie précédemment.

La présente invention a également pour objet des moyens pour traiter la dépendance à l'alcool chez un être humain par l'administration 1 à 4 fois par jour d'une composition pharmaceutique telle que de définie précédemment.

La présente invention a également pour des moyens pour traiter la dépendance à l'alcool chez un être humain par l'administration journalière 1 à 7 jours par semaine d'une composition pharmaceutique telle que de définie précédemment, l'administration pouvant ou non s'effectuer selon un calendrier continu.

Les deux principes actifs constituant la nouvelle composition pharmaceutique selon l'invention peuvent être administrés sous la forme d'une composition pharmaceutique unitaire comprenant les deux principes actifs permettant une administration de ladite composition au patient en une seule prise.

Néanmoins, une administration séparée des deux principes actifs constitutifs de la nouvelle composition pharmaceutique selon l'invention peut également être envisagée. Ainsi, la présente invention a également pour objet un produit pharmaceutique contenant :
- un composé ayant une action antagoniste sur les récepteurs 5-HT2 sérotoninergiques choisi comme étant la cyproheptadine ; et
- un composé ayant une action antagoniste sur les récepteurs alpha1-noradrénergiques choisi parmi la prazosine, l'alfuzosine, la térazosine et la tamsulosine; comme produit de combinaison (ou kit pharmaceutique) pour une administration simultanée, séparée ou étalée dans le temps dans le cadre du traitement de la dépendance à l'alcool chez l'être humain.

Le produit pharmaceutique selon l'invention peut bien entendu être administré selon l'un des schémas d'administration définis précédemment. Ainsi, la présente invention a également pour objet un produit pharmaceutique contenant :
- un composé ayant une action antagoniste sur les récepteurs 5-HT2 sérotoninergiques choisi comme étant la cyproheptadine ; et
- un composé ayant une action antagoniste sur les récepteurs alpha 1-noradrénergiques choisi parmi la prazosine, l'alfuzosine, la térazosine et la tamsulosine; comme produit de combinaison (ou kit pharmaceutique) pour une administration simultanée, séparée ou étalée dans le temps 1 à 4 fois par jour, dans le cadre du traitement de la dépendance à l'alcool chez l'être humain.

Le produit pharmaceutique selon l'invention peut bien entendu être administré selon l'un des schémas d'administration définis précédemment. Ainsi, la présente invention a également pour objet un produit pharmaceutique contenant :
- un composé ayant une action antagoniste sur les récepteurs 5-HT2 sérotoninergiques choisi comme étant la cyproheptadine ; et
- un composé ayant une action antagoniste sur les récepteurs alpha1-noradrénergiques choisi parmi la prazosine, l'alfuzosine, la térazosine et la tamsulosine; comme produit de combinaison (ou kit pharmaceutique) pour une administration journalière simultanée, séparée ou étalée dans le temps 1 à 7 jours par semaine dans le cadre du traitement de la dépendance à l'alcool chez l'être humain.

La présente invention est illustrée de manière non limitative par les exemples suivants.

### Exemple 1

### Matériel

Le modèle utilisé est la consommation d'alcool chez la souris mâle de la lignée C57BL/6J, qui présente une appétence importante pour l'alcool. Le protocole utilisé est comparable à ceux utilisés dans de précédentes études (Grahame et al., 2000, Naltrexone and alcohol drinking in mice lacking β-endorphin by site-directed mutagenesis, Pharmacol Biochem Behav ; 67 ; pp 759-766 ; Finn et al., 2005, A procedure to produce high alcohol intake in mice ; Psychopharmacology 178 ; pp 471-480 ; Rhodes et al., 2005, Evaluation of a simple model of ethanol drinking to intoxication in C57BL/6J mice, Physiol Behav ; 54 ; pp 53-63).

### Produits

Tous les produits utilisés pour les traitements sont mis en solution dans du sérum physiologique (NaCl 0,9%) de façon à ce que le volume d'injection soit de 10 ml / kg de poids corporel.

La prazosine a été testé aux doses suivantes : 1 mg/kg ; 0,5 mg/kg.
La cyproheptadine a été testée à la dose suivante : 1 mg/kg
L'ifenprodil a été testé à la dose suivante : 1 mg/kg

### Protocole de mesure de la consommation d'alcool

Les animaux sont placés dans leur cage d'hébergement pendant toute la durée de l'expérience. Ils sont soumis à une alcoolisation forcée, avec de l'alcool à 10% comme seule boisson, pendant 15 jours. Ils sont ensuite remis dans des conditions normales avec de l'eau pour boisson.

Différents groupes de souris sont constitués en fonction des traitements : témoins (injectés avec du saline), prazosine 1 mg/kg, cyproheptadine 1 mg/kg, prazosine 1 mg/kg + cyproheptadine 1 mg/kg, prazosine 0,5 mg/kg + cyproheptadine 1 mg/kg,

Les tests débutent 24 heures après l'arrêt de l'alcoolisation forcée. Ils se déroulent pendant les deux premières heures d'obscurité (période d'activité), durant lesquelles les animaux ont accès à un biberon d'eau et un biberon d'alcool. Les quantités d'eau et d'alcool consommées sont mesurées après ces deux heures.

Plusieurs sessions de mesure de consommation d'eau et d'alcool sont réalisées. Pendant les 2 premières sessions (S 1-S2) les animaux reçoivent une injection de saline, ils reçoivent les traitements (prazosine, cyproheptadine, prazosine + cyproheptadine) ou saline pour le groupe témoin) aux séances suivantes (T1, T2, T3). Les traitements sont effectués 10 minutes avant chaque session.

L'indice de préférence Alcool/Eau (Pref.) est calculé de la manière suivante
Pref. = (Consommation Alcool - Eau) / (Consommation Alcool + Eau)
0 < Pref. ≤ 1 : Préférence pour l'alcool
Pref. = 0 : Pas de préférence alcool vs eau
-1 ≤ Pref. < 0 : Préférence pour l'eau (i.e. aversion pour l'alcool)

Lors des séances de mesure de consommation d'alcool, les animaux reçoivent les traitements 30 minutes avant ladite mesure.

Les résultats sont exprimés en valeur moyenne et erreur standard à la moyenne (ESM). Les indices de préférence (Pref) des groupes traités sont comparés à celui du groupe témoin par le test de t de Student bilatéral pour groupes indépendants. Les valeurs indiquées (p vs Tem) sont les probabilités pour que la différence entre groupe traité et groupe témoin soit due au hasard. Une différence est considérée comme statistiquement significative si p ≤ 0.05.

### Résultats

### Expérience A

**Tableau A- Effet d'un traitement par la cyproheptadine et la prazosine sur la préférence alcoolique. (n = 6 animaux par groupe)**

| | | S-2 | S-1 | T1 | T2 | T3 |
|---|---|---|---|---|---|---|
| Témoin | **Moyenne** | **0,69** | **0,86** | **0,67** | **0,94** | **1,00** |
| n=6 | ESM | 0,14 | 0,14 | 0,15 | 0,06 | 0,00 |
| | | | | | | |
| Cyproheptadine (1 mg/kg) | **Moyenne** | **0,84** | **0,78** | **0,92** | **0,82** | **0,82** |
| n=6 | ESM | 0,10 | 0,10 | 0,08 | 0,12 | 0,12 |
| | p vs Tem | 0,43 | 0,66 | 0,17 | 0,37 | 0,16 |
| | | | | | | |
| Prazosine (1 mg/kg) | **Moyenne** | **0,68** | **0,61** | **0,78** | **0,57** | **0,72** |
| n=6 | ESM | 0,12 | 0,13 | 0,22 | 0,15 | 0,18 |
| | p vs Tem | 0,93 | 0,24 | 0,69 | 0,25 | 0,16 |

Pref. = (Consommation Alcool - Eau) / (Consommation Alcool + Eau)
S2 et S1 : séances de traitement saline pour tous les groupes
T1, T2 et T3 : séances de traitement par la cyproheptadine 1 mg/kg ou la prazosine 1 mg/kg, les témoins recevant du saline.

Ces résultats indiquent que, sur ce modèle et aux doses utilisées, la cyproheptadine et la prazosine administrés seuls n'ont pas d'effet significatif sur la préférence alcoolique.

### Expérience B

**Tableau B- Effet d'un traitement par la cyproheptadine en combinaison avec la prazosine sur la Préférence alcoolique (n = 6 ou 9 animaux par groupe)**

| | | S-2 | S-1 | T1 | T2 | T3 |
|---|---|---|---|---|---|---|
| Témoin | **Moyenne** | **0,69** | **0,86** | **0,94** | **1,00** | **1,00** |
| n=6 | ESM | 0,14 | 0,14 | 0,06 | 0,00 | 0,00 |
| | | | | | | |
| Cyproheptadine (1 mg/kg) / Prazosine (0,5 mg/kg) | **Moyenne** | **0,81** | **0,94** | **-0,37** | **-0,70** | **-0,81** |
| n=9 | ESM | 0,10 | 0,06 | 0,21 | 0,15 | 0,13 |
| | p vs Tem | 0,62 | 0,52 | 0,0002 | 0,0001 | 0,0001 |
| | | | | | | |
| Cyproheptadine (1 mg/kg) / Prazosine (1 mg/kg) | **Moyenne** | **0,79** | **0,82** | **-0,25** | **-0,56** | **-0,83** |
| n=6 | ESM | 0,11 | 0,12 | 0,36 | 0,20 | 0,17 |
| | p vs Tem | 0,62 | 0,85 | 0,04 | 0,0001 | 0,0001 |

Pref. = (Consommation Alcool - Eau) / (Consommation Alcool + Eau)
S2 et S1 : séances traitement de saline
T1, T2 et T3 : séances de traitement par la cyproheptadine 1 mg/kg en combinaison avec la prazosine 0,5 mg/kg ou 1 mg/kg. Le groupe Témoin reçoit une administration de saline, comme aux séances S1 et S2.

Ces résultats indiquent que, sur ce modèle et à ces doses, la cyproheptadine et la prazosine administrés en combinaison ont un effet significatif sur la préférence alcoolique. On notera que cette combinaison entraine une forte aversion pour l'alcool (Préférence négative et se rapprochant de -1), quelle que soit la dose de prazosine.

Une synergie d'action entre la cyproheptadine et la prazosine est donc mise en évidence.

### Expérience C

**Tableau C - Effet d'un traitement par la cyproheptadine en combinaison avec l'ifenprodil sur la préférence alcoolique (n = 6 ou 9 animaux par groupe)**

| | | S1 | S2 | T1 | T2 | T3 |
|---|---|---|---|---|---|---|
| Témoins (n=12) | **Moyenne** | **0,91** | **0,92** | **0,95** | **0,96** | **0,92** |
| | ESM | 0,070 | 0,054 | 0,360 | 0,042 | 0,056 |
| Ifenprodil (1 mg/kg) + Cyproheptadine (1 mg/kg) | **Moyenne** | **0,93** | **0,95** | **0,75** | **0,08** | **0,00** |
| | ESM | 0,067 | 0,950 | 0,123 | 0,104 | 0,089 |
| | p vs Tem | 0.79564 | 0,72327 | 0,06208 | 0,00000009 | 0,000000014 |

Pref= (Consommation Alcool - Eau) / (Consommation Alcool + Eau)
S2 et S1 : séances traitement saline
T1, T2 et T3 : séances traitement cyproheptadine 1 mg/kg en combinaison avec l'ifenprodil 1 mg/kg. Le groupe Témoin reçoit une administration de saline, comme aux séances S1 et S2.

Ces résultats indiquent que :
- le traitement par une composition ifenprodil + cyproheptadine est efficace pour réduire la consommation d'alcool dans ce modèle, cet effet augmentant au fur et mesure des séances ;
- mais qu'en comparaison avec le traitement par une composition cyproheptadine + prazosine, la composition cyproheptadine + ifenprodil est moins efficace, celle-ci ne déclenchant pas une aversion telle que l'on peut la voir avec les valeurs négatives de P induites par la combinaison cyproheptadine/prazosine (voir les résultats de l'exemple B ci-avant).

### Conclusion

La prazosine et la cyproheptadine administrées séparément n'ont pas d'effet sur la consommation d'alcool, tandis que le même modèle et aux mêmes doses, la prazosine et la cyproheptadine administrés en combinaison réduisent de manière significative la consommation d'alcool, ce qui est caractéristique d'une synergie entre les composés.

La prazosine et la cyproheptadine, administrées en combinaison à des doses qui n'ont pas d'effet incapacitant majeur, réduisent de manière très significative la préférence pour l'alcool, jusqu'à déclencher une aversion. Cet effet est supérieur aux effets de la combinaison ifenprodil et cyproheptadine décrite comme efficace dans des modèles de dépendance aux psychostimulants dans la demande de brevet WO 2006/018538.

De plus, l'effet de la composition prazosine/cyproheptadine sur la consommation alcoolique augmente au fur et à mesure des administrations, et ce, quelles que soient les doses utilisées. Ce résultat permet de considérer favorablement un traitement chronique, avec une possibilité de moduler, voire limiter les doses.

Ces résultats indiquent enfin que la composition prazosine/cyproheptadine peut contrecarrer les effets appétitifs des différentes substances addictives, et par voie de conséquence réduire les états de dépendance.

### Exemple 2

Les associations cyproheptadine/alfuzosine, cyproheptadine/térazosine et cyproheptadine/tamsulosine ont été testées dans des conditions expérimentales identiques à celles décrites dans l'exemple 1 précédent et en utilisant le même modèle.

Les résultats obtenus sont rapportés dans le Tableau D suivant.

**Tableau D - Effet d'un traitement par la cyproheptadine en combinaison avec l'alfuzosine, la térazosine ou la tamsulosine sur la préférence alcoolique (n = 6 ou 9 animaux par groupe)**

| | | S-2 | S-1 | **T1** | **T2** | **T3** |
|---|---|---|---|---|---|---|
| Témoin (n=6) | Moyenne | 0,54 | 0,63 | **0,72** | **0,84** | **0,97** |
| Cyproheptadine (1 mg/kg) (n=9) | Moyenne | 0.66 | 0,68 | **0,91** | **0,76** | **0,81** |
| Alfuzosine (0,5 mg/kg) (n=9) | Moyenne | 0,72 | 0,74 | **0,59** | **0,62** | **0,63** |
| Térazosine (0,5 mg/kg) (n=9) | Moyenne | 0,65 | 0,81 | **0,64** | **0,75** | **0,72** |
| Tamsulosine (0,5 mg/kg) (n=9) | Moyenne | 0,71 | 0,73 | **0,68** | **0,79** | **0,77** |
| Cyproheptadine (1 mg/kg) + Alfuzosine (0,5 mg/kg) (n=9) | Moyenne | 0,83 | 0,91 | **-0,48** | **-0,84** | **-0,87** |
| Cyproheptadine (1 mg/kg) + Térazosine (0,5 mg/kg) (n=9) | Moyenne | 0,68 | 0,83 | **-0,13** | **-0,68** | **-0,76** |
| Cyproheptadine (1 mg/kg) + Tamsulosine (0,5 mg/kg) (n=9) | Moyenne | 0,74 | 0,87 | **-0,17** | **-0,45** | **-0,73** |

Pref = (Consommation d'alcool - eau)/(Consommation d'alcool + eau)
S-1 et S-2 = traitement avec une solution saline pour tous les groupes
T1, T2 et T3 = traitement avec produits ; le groupe témoin recevant une solution saline

### Conclusion

La cyproheptadine, l'alfuzosine, la térazosine et la tamsulosine administrées séparément n'ont pas d'effet sur la consommation d'alcool, tandis que sur le même modèle et aux mêmes doses, les associations cyproheptadine/alfuzosine, cyproheptadine/térazosine et cyproheptadine/tamsulosine réduisent de manière significative la consommation d'alcool, ce qui est caractéristique d'une synergie entre les composés.

Cet effet est supérieur aux effets de la combinaison ifenprodil et cyproheptadine décrite comme efficace dans des modèles de dépendance aux psychostimulants dans la demande de brevet WO 2006/018538 (voir l'exemple 1 précédent).

Ces résultats indiquent également que, sur ce modèle et à ces doses, les associations cyproheptadine/alfuzosine, cyproheptadine/térazosine et cyproheptadine/tamsulosine ont un effet significatif sur la préférence alcoolique. On notera que ces combinaisons entrainent une forte aversion pour l'alcool (Préférence négative et se rapprochant de -1), quelle que soit l'association.

Une synergie d'action entre la cyproheptadine et l'alfuzosine, la térazosine ou la tamsulosine est mise en évidence.

## Revendications

1. Composition pharmaceutique pour le traitement de la dépendance à l'alcool chez l'être humain comprenant deux principes actifs :
- un composé ayant une action antagoniste sur les récepteurs 5-HT2 sérotoninergiques choisi comme étant la cyproheptadine ; et
- un composé ayant une action antagoniste sur les récepteurs alpha1-noradrénergiques choisi parmi la prazosine, l'alfuzosine, la térazosine et la tamsulosine.

2. Composition selon la revendication 1, **caractérisée en ce que** le composé ayant une action antagoniste sur les récepteurs alpha1-noradrénergiques est la prazosine.

3. Composition pharmaceutique selon la revendication 1 ou 2 **caractérisée en ce qu'**elle comprend :
- de 0,04 à 20 mg de cyproheptadine; et
- de 0,025 à 20 mg de prazosine, de 0,075 à 10 mg d'alfuzosine, de 0,01 à 5 mg de térazosine ou de 0,004 à 0,4 mg de tamsulosine.

4. Composition pharmaceutique selon l'une des revendications 1 à 3 pour une administration 1 à 4 fois par jour au patient dépendant à l'alcool.

5. Composition pharmaceutique selon l'une des revendications 1 à 4 pour une administration journalière 1 à 7 jours par semaine au patient dépendant à l'alcool.

6. Produit pharmaceutique contenant :
- un composé ayant une action antagoniste sur les récepteurs 5-HT2 sérotoninergiques choisi comme étant la cyproheptadine ; et
- un composé ayant une action antagoniste sur les récepteurs alpha1-noradrénergiques choisi parmi la prazosine, l'alfuzosine, la térazosine et la tamsulosine; comme produit de combinaison pour une administration simultanée, séparée ou étalée dans le temps dans le cadre du traitement de la dépendance à l'alcool chez l'être humain.

## Patentansprüche

1. Pharmazeutische Zusammensetzung zur Behandlung von Alkoholabhängigkeit beim Menschen, die zwei Wirkstoffe umfasst:
- eine Verbindung mit einer antagonistischen Wirkung auf die serotoninergen 5-HT2-Rezeptoren, die als Cyproheptadin ausgewählt ist; und
- eine Verbindung mit einer antagonistischen Wirkung auf die noradrenergen Alpha-1-Rezeptoren, die aus Prazosin, Alfuzosin, Terazosin und Tamsulosin ausgewählt ist.

2. Zusammensetzung nach Anspruch 1, **dadurch gekennzeichnet, dass** die Verbindung mit einer antagonistischen Wirkung auf die noradrenergen Alpha-1-Rezeptoren Prazosin ist.

3. Pharmazeutische Zusammensetzung nach Anspruch 1 oder 2, **dadurch gekennzeichnet, dass** sie Folgendes umfasst:
- 0,04 bis 20 mg Cyproheptadin und
- 0,025 bis 20 mg Prazosin, 0,075 bis 10 mg Alfuzosin, 0,01 bis 5 mg Terazosin oder 0,004 bis 0,4 mg Tamsulosin.

4. Pharmazeutische Zusammensetzung nach einem der Ansprüche 1 bis 3 für eine Verabreichung 1- bis 4-mal täglich an den alkoholabhängigen Patienten.

5. Pharmazeutische Zusammensetzung nach einem der Ansprüche 1 bis 4 für eine tägliche Verabreichung an 1 bis 7 Tagen in der Woche an den alkoholabhängigen Patienten.

6. Pharmazeutisches Produkt, das Folgendes enthält:
- eine Verbindung mit einer antagonistischen Wirkung auf die serotoninergen 5-HT2-Rezeptoren, die als Cyproheptadin ausgewählt ist; und
- eine Verbindung mit einer antagonistischen Wirkung auf die noradrenergen Alpha-1-Rezeptoren, die aus Prazosin, Alfuzosin, Terazosin und Tamsulosin ausgewählt ist;
als Kombinationsprodukt für eine gleichzeitige, getrennte oder zeitlich versetzte Verabreichung im Rahmen der Behandlung der Alkoholabhängigkeit beim Menschen.

## Claims

1. A pharmaceutical composition for the treatment of alcohol addiction in human beings comprising two active ingredients:
- a compound having an antagonistic activity on the serotoninergic 5-HT2 receptors selected as the cyproheptadine; and
- a compound having an antagonistic activity on the alpha1-noradrenergic receptors selected from prazosin, alfuzosin, terazosin and tamsulosin

2. The composition according to claim 1, **characterized in that** the compound having an antagonistic activity on the alpha1-noradrenergic receptors is prazosin.

3. The pharmaceutical composition according to claim 1 or 2 **characterized in that** it comprises :
- from 0.04 to 20 mg of cyproheptadin; and
- from 0.025 to 20 mg of prazosin, from 0.075 to 10 mg of alfuzosin, from 0.01 to 5 mg of terazosin or from 0.004 to 0.4 mg of tamsulosin.

4. The pharmaceutical composition according to any of claims 1 to 3 for an administration of 1 to 4 times a day to an alcohol addicted patient.

5. The pharmaceutical composition according to any of claims 1 to 4 for a daily administration of 1 to 7 days a week to an alcohol addicted patient.

6. A pharmaceutical product containing:
- a compound having an antagonistic activity on the serotoninergic 5-HT2 receptors selected as the cyproheptadine; and
- a compound having an antagonistic activity on the alpha1-noradrenergic receptors selected from prazosin, alfuzosin, terazosin and tamsulosin; as a combination product for a simultaneous, separated or spread out over time administration for the treatment of alcohol addiction in human beings.
